# EUROPÄISCHE PATENTSCHRIFT

(11) **EP 2 283 789 B1**
(45) Veröffentlichungstag und Bekanntmachung des Hinweises auf die Patenterteilung: **12.09.2012**
(21) Anmeldenummer: 10170148.0
(22) Anmeldetag: 20.07.2010
(51) Int. Cl.: A61B 19/00

(54) **Verbindungsvorrichtung zur Verwendung in einem Reinigungsraum in einer Reinigungsvorrichtung zur Reinigung von chirurgischen Instrumenten**
Connection device for use in a cleaning room in a cleaning device for cleaning surgical instruments
Dispositif de liaison pour l'utilisation dans une pièce de nettoyage, dans un dispositif de nettoyage, pour le nettoyage d'instruments chirurgicaux

(30) Priorität: 10.08.2009 DE 102009036564
(43) Veröffentlichungstag der Anmeldung: 16.02.2011
(73) Patentinhaber: OLYMPUS WINTER & IBE GMBH, 22045 Hamburg (DE)
(72) Erfinder: Eschborn, Sascha, 22926, Ahrensburg (DE)
(74) Vertreter: Seemann, Ralph

(56) Entgegenhaltungen:
- EP-A1- 0 878 165
- EP-A1- 1 952 782
- DE-B3- 10 321 991

## Beschreibung

Die Erfindung betrifft eine Verbindungsvorrichtung zur Verwendung in einem Reinigungsraum einer Reinigungsvorrichtung zur Reinigung von chirurgischen Instrumenten, insbesondere Endoskopen. Die Erfindung betrifft ferner eine entsprechende Anordnung dieser Verbindungsvorrichtung in einem Reinigungsraum einer Reinigungsvorrichtung sowie die Verwendung einer entsprechenden Verbindungsvorrichtung.

Entsprechende Verbindungsvorrichtungen sind bekannt. Hierbei handelt es sich üblicherweise um Schläuche, die dazu vorgesehen sind, um beispielsweise Wasser, das durch eine Reinigungsvorrichtung zur Verfügung gestellt wird, in ein chirurgisches Instrument, beispielsweise ein Endoskop, zu leiten, um dort einen Kanal in dem Endoskop zu spülen.

Aus DE 39 18 432 C2 ist ein Verfahren zum Desinfizieren von Endoskopen und ein Sterilisator zur Durchführung des Verfahrens bekannt. Hierbei wird überprüft, ob ausreichend Spülflüssigkeit durch die Endoskopkanäle hindurchgespült wird.

Aus EP 1 477 106 A1 ist auch eine Reinigungsvorrichtung bekannt, bei der auf einem Tablett ein Endoskop eingebracht wird, wobei entsprechend Zuführschläuche in der Reinigungsvorrichtung vorgesehen sind, die mit Kanälen des Endoskops verbunden werden können.

Aus EP 0 709 056 B1 ist eine Vorrichtung zum Reinigen und Sterilisieren von Endoskopen bekannt, wobei in der Reinigungsvorrichtung eine Pumpe vorgesehen ist, die zur Druckerhöhung vorgesehen ist.

EP 0 878 165 A1 offenbart eine Vorrichtung und ein Verfahren zum Reinigen und/oder Desinfizieren von Endoskopen. Es ist eine Druckverteilungsvorrichtung vorgesehen und außerdem eine Pumpe.

DE 103 21 991 B3 offenbart eine Vorrichtung zum Reinigen und/oder Desinfizieren von langgestreckten Hohlkörpern, insbesondere von medizinischen Schläuchen und Kathetern, wobei in entsprechenden Leitungen eine Strömungsverengung und ein Drucksensor vorgesehen sind.

EP 1 952 782 A1 offenbart ein System mit Adapter zur Reinigung von medizinischen Geräten. Es ist eine Kupplungseinrichtung, die aus Einwegkupplungen ausgebildet ist, vorgesehen, die nach einem erstmaligen Ankuppeln einer medizinischen Vorrichtung beim Abkuppeln ein Leck im Fluidkanal erzeugt.

Es gibt Kanäle in chirurgischen Instrumenten, insbesondere Endoskopkanäle, die zur Reinigung oder Desinfektion mit erhöhtem Wasserdruck beaufschlagt werden müssen. Entsprechende Kanäle sind beispielsweise Albarankanäle oder treten bei Duodenoskopen auf. Üblicherweise in Kliniken vorgesehene Reinigungsvorrichtungen weisen keine Wasserzufuhr mit erhöhtem Wasserdruck auf. Stattdessen wird Wasser oder eine andere Flüssigkeit, beispielsweise desinfiziertes Wasser, das mit einer Reinigungslösung versetzt ist, durch eine übliche Umwälzpumpe bei bis zu 1,2 bar durch die Reinigungsvorrichtung gefördert.

Es ist Aufgabe der vorliegenden Erfindung, eine variable und kostengünstige Möglichkeit bei üblichen Reinigungsmaschinen bzw. Aufbereitungsmaschinen vorzusehen, bei der wenigstens ein Spülkreislauf variabel an Gegebenheiten der zu reinigenden Kanäle angepasst werden kann.

Gelöst wird diese Aufgabe durch eine Verbindungsvorrichtung zur Verwendung in einem Reinigungsraum einer Reinigungsvorrichtung zur Reinigung von chirurgischen Instrumenten, insbesondere Endoskopen, wobei die Verbindungsvorrichtung ein Gehäuse mit einem Eingangsanschluss und einem Ausgangsanschluss aufweist, wobei der Eingangsanschluss mit einem Anschluss der Reinigungsvorrichtung, mittels dessen ein flüssiges Medium bereitgestellt wird, verbindbar oder verbunden ist, wobei der Ausgangsanschluss mit einem zu reinigenden Kanal des chirurgischen Instruments verbindbar oder verbunden ist, wobei in dem Gehäuse ein Mittel zur Beeinflussung wenigstens einer physikalischen Eigenschaft und/oder chemischen Eigenschaft des flüssigen Mediums vorgesehen ist, wobei in dem Gehäuse (1) eine Pumpe (11) zur Veränderung des Drucks des an dem Eingangsanschluss (4) zur Verfügung gestellten Mediums vorgesehen ist, wobei die Pumpe (11) den Druck erhöht, wobei in dem Gehäuse (1) eine autonome Spannungsversorgung (8) vorgesehen ist, um die Pumpe (11) mit Spannung zu versorgen.

Durch Vorsehen einer Verbindungsvorrichtung mit einem Gehäuse, durch das ein flüssiges Medium, das von einer Reinigungsvorrichtung bereitgestellt wird, hindurchfließen kann und einem zu reinigenden Kanal des chirurgischen Instruments zugeführt werden kann, ist es möglich, auf einfache Art und Weise die Gegebenheiten bzw. die physikalischen und/oder chemischen Eigenschaften des Mediums an den zu reinigenden Kanal anzupassen. So kann beispielsweise eine gesonderte Spülsteuerung durch ein gesondert in dem Gehäuse angeordnetes Ventil, durch das das Medium fließt, eingestellt werden. Ferner kann eine Temperaturänderung durch Vorsehen einer Heizvorrichtung und/oder Kühlvorrichtung in dem Gehäuse der Verbindungsvorrichtung vorgenommen werden. Das Mittel zur Beeinflussung der wenigstens einen physikalischen Eigenschaft des flüssigen Mediums ist dann die Heizvorrichtung und/oder die Kühlvorrichtung.

Erfindungsgemäß ist in dem Gehäuse eine Pumpe zur Veränderung des Drucks des an dem Eingangsanschluss zur Verfügung gestellten Mediums vorgesehen. Das Mittel zur Beeinflussung der wenigsten einen physikalischen Eigenschaft des flüssigen Mediums ist in diesem Fall die Pumpe. Der veränderte Druck wird dann am Ausgangsanschluss zur Verfügung gestellt und gelangt durch das chirurgische Instrument bzw. einen Kanal im chirurgischen Instrument. Die Pumpe erhöht erfindungsgemäß den Druck, insbesondere auf 1,5 bis 4 bar, vorzugsweise insbesondere auf 2 bis 3 bar. Dieser Druck wird dann auch am Ausgangsanschluss zur Verfügung gestellt. Vorzugsweise können mittels der Pumpe auch Druckstöße erzeugt werden, beispielsweise durch Vorsehen eines Schalters an der Stromversorgung der Pumpe oder durch Vorsehen von Spannungsvariationen der Spannung, die die Pumpe versorgt.

Vorzugsweise ist ein Reservoir für das flüssige Medium in dem Gehäuse stromaufwärts der Pumpe vorgesehen. Dieses Reservoir, das auch als Zwischenspeicher bezeichnet werden kann, dient dazu, gleichmäßig das flüssige Medium der Pumpe zur Verfügung zu stellen. Insbesondere kann das Reservoir auch als Puffer dienen, um Zufuhrschwankungen des zugeführten Mediums, das am Eingangsanschluss der Verbindungsvorrichtung zugeführt wird, auszugleichen.

Vorzugsweise ist der Querschnitt des Reservoirs größer als der Querschnitt des Eingangsanschlusses. Bei dem Querschnitt ist hier insbesondere der freie Querschnitt, also der Querschnitt, der von der Innenwandung des Reservoirs bzw. des Eingangsanschlusses umschlossen ist, gemeint.

Vorzugsweise weist das Reservoir ein Volumen zwischen 100 ml und 10 I, insbesondere vorzugsweise zwischen 500 ml und 5 I und insbesondere vorzugsweise zwischen 1 und 2 I, auf.

Vorzugsweise ist in dem Gehäuse ein Drucksensor vorgesehen. Der Drucksensor misst vorzugsweise den Druck des Mediums im Reservoir oder in der Nähe des Reservoirs. Ferner vorzugsweise steuert der Drucksensor die Pumpe bzw. die Leistung der Pumpe. Insbesondere vorzugsweise ist der Drucksensor ein Druckschalter oder in einem Druckschalter integriert, der unmittelbar den Betrieb der Pumpe schaltet.

Beispielsweise ist es möglich, die Pumpe ab einem vorgebbaren Grenzdruck von beispielsweise 1 bar oder etwas über 1 bar in Betrieb zu setzen. In diesem Fall pumpt die Pumpe nur dann, wenn eine in der Reinigungsvorrichtung vorgesehene Umwälzpumpe pumpt bzw. ausreichend Druck in der Leitung des flüssigen Mediums, beispielsweise der Wasserleitung, vorherrscht und entsprechend dann gereinigtes Wasser bzw. ein gereinigtes Medium oder Fluid der Verbindungsvorrichtung zur Verfügung gestellt wird. Anstelle eines Drucksensors können auch elektrische Signale, die aus der Reinigungsvorrichtung übermittelt werden, beispielsweise um anzuzeigen, dass die Umwälzpumpe zur Erzeugung eines Fluid- bzw. Mediumkreislaufes in Betrieb ist, die Pumpe in der Verbindungsvorrichtung steuern. Da die Verbindungsvorrichtung vorzugsweise im Reinigungsraum der Reinigungsvorrichtung angeordnet ist, also selbst mit dem flüssigen Medium bzw. mit einem Fluid wie beispielsweise gereinigtes Wasser in Verbindung kommt, könnte auch ein elektromagnetisches Signal von der Reinigungsvorrichtung zur Verfügung gestellt werden, um die Pumpe in der Verbindungsvorrichtung zu steuern.

Erfindungsgemäß ist in dem Gehäuse eine autonome Spannungsversorgung, insbesondere eine Batterie oder ein Akkumulator, vorgesehen, insbesondere um die Pumpe mit Spannung zu versorgen. Hierdurch ist eine sehr flexible und einfach zu benutzende Verbin-dungsvorrichtung möglich.

Vorzugsweise ist das Gehäuse wasserdicht.

Die Aufgabe wird ferner durch eine Anordnung einer erfindungsgemäßen Verbindungsvorrichtung in einem Reinigungsraum einer Reinigungsvorrichtung für chirurgische Instrumente, insbesondere Endoskope, gelöst. Hierdurch können sehr effizient und sehr variabel chirurgische Instrumente, insbesondere Endoskope, mit beispielsweise einem Albarankanal entsprechend mit den üblichen Reinigungsvorrichtungen, die ein flüssiges Medium mit nur geringem Druck zur Verfügung stellen, auch gereinigt werden.

Vorzugsweise ist die Verbindungsvorrichtung eingangsseitig mit einem Mediumsanschluss der Reinigungsvorrichtung verbunden und ausgangsseitig mit einem im Reinigungsraum angeordneten chirurgischen Instrument verbunden. Diese Verbindung geschieht vorzugsweise über Rohre oder Schläuche. Die Aufgabe wird ferner durch die Verwendung einer erfindungsgemäßen Verbindungsvorrichtung zum, insbesondere druckerhöhten, Durchleiten eines flüssigen Mediums durch die Verbindungsvorrichtung und Einleiten des, insbesondere druckerhöhten, flüssigen Mediums in ein chirurgisches Instrument gelöst.

Ferner wird die Aufgabe durch einen Reinigungsbehälter mit einer integrierten erfindungsgemäßen Verbindungsvorrichtung gelöst.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische dreidimensionale Darstellung einer Reinigungsvorrichtung mit einer erfindungsgemäßen Verbindungsvorrichtung,
- Fig. 2: eine schematische Schnittdarstellung durch eine erfin- dungsgemäße Verbindungsvorrichtung,
- Fig. 3: eine schematische Draufsicht auf einen erfindungsge- mäßen Reinigungsbehälter und
- Fig. 4: eine schematische Darstellung der Integration einer er- findungsgemäßen Verbindungsvorrichtung in einer Rei- nigungsvorrichtung.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

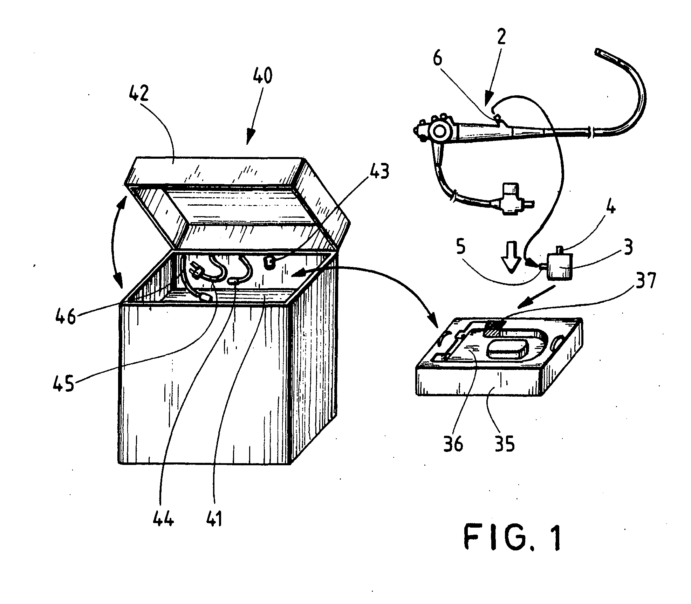

Fig. 1 zeigt eine Reinigungsvorrichtung 40, die als Endoskopreinigungsvorrichtung ausgebildet ist. Die Endoskopreinigungsvorrichtung 40 weist einen Innenbereich in Form eines Reinigungsbads 41 auf, in das ein Reinigungsbehälter 35 eingebracht werden kann. Es ist ferner ein Deckel 42 vorgesehen, der wasserdicht verschlossen werden kann.

In dem Reinigungsbad 41 ist eine Wasserzuführung 43 vorgesehen, die Wasser in das Reinigungsbad 41 bzw. nach Einbringen des Reinigungsbehälters 35 in den Aufnahmebereich 36 des Reinigungsbehälters 35 einbringen kann. Es sind außerdem eine Desinfektionsmittelzuführung 44, eine Luft- und/oder Wasserzuführung 45 und eine Wasserabsaugung 46 vorgesehen. Die entsprechenden Leitungen 43 bis 46 können teilweise mit dem Endoskop 2, der erfindungsgemäßen Verbindungsvorrichtung 3 verbunden sein und/oder in dem Aufnahmebereich 36 für das Endoskop 2 enden.

Wie der Doppelpfeil andeutet, kann der Reinigungsbehälter 35 in das Reinigungsbad 41 eingebracht werden. Anstelle eines Reinigungsbades 41 kann auch eine Reinigungsdusche vorgesehen sein. In dem Aufnahmebereich 36 des Reinigungsbehälters 35 ist eine Aussparung 37 vorgesehen, in die die erfindungsgemäße Verbindungsvorrichtung 3 eingebracht werden kann.

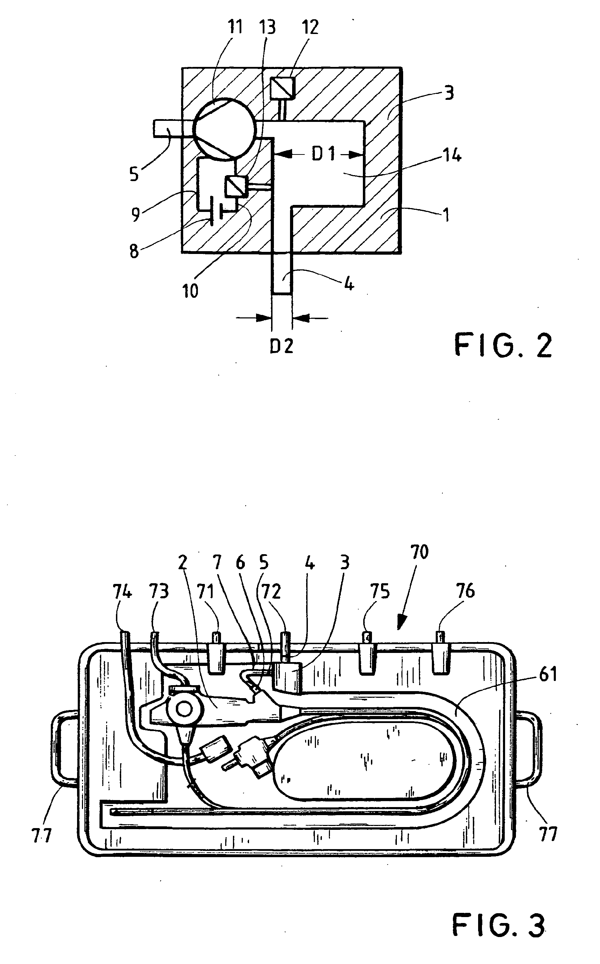

Die Verbindungsvorrichtung 3 weist einen Anschluss 4 auf, der als Eingangsanschluss ausgebildet ist und beispielsweise mit der Desinfektionsmittelzuführung 44 verbunden werden kann. Außerdem weist die erfindungsgemäße Verbindungsvorrichtung einen Ausgangsanschluss 5 auf, der mit beispielsweise dem Anschluss 6 des Endoskops 2 verbunden werden kann. Das Endoskop 2 wird dann auch in den Aufnahmebereich 36 eingebracht. Ein entsprechend eingebrachtes Endoskop 2 ist beispielsweise in Fig. 3 dargestellt, wobei der Reinigungsbehälter 70 der Fig. 3 etwas anders ausgestaltet ist als der Reinigungsbehälter 35 aus Fig. 1. Gleichwohl wird das Endoskop 2 entsprechend auch in dem Reinigungsbehälter 35 der Fig. 1 eingebracht.

Die erfindungsgemäße Verbindungsvorrichtung 3 ist schematisch in einer Ausführungsform in einer Schnittdarstellung in Fig. 2 dargestellt. Es ist entsprechend ein Gehäuse 1 vorgesehen, in das eine Pumpe 11 und ein Akkumulator 8 eingebracht sind, wobei der Akkumulator 8 die Spannung für die Pumpe 11 zur Förderung bzw. zur Druckerhöhung eines Mediums, insbesondere eines Fluids, wie beispielsweise gereinigtes oder desinfiziertes Wasser oder mit einem Desinfektionsmittel versetztes Wasser, bereitstellt.

Es sind zwei Drucksensoren 12 und 13 vorgesehen, wobei der Drucksensor 13 als Druckschalter ausgestaltet ist. Die beiden Drucksensoren 12 und 13 können alternativ vorgesehen sein. Der Druckschalter 13 kann beispielsweise, was sehr schematisch dargestellt ist, das Kabel 10 bzw. die Leitung 10 unterbrechen, so dass die Pumpe 11 nicht fördert bzw. nicht den Druck erhöht. Entsprechend ist das Kabel 9 ununterbrochen dargestellt. Der Druckschalter 13 kann beispielsweise so ausgestaltet sein, dass bei einem gemessenen Druck in dem Reservoir 14 von 1 bar oder etwas über 1 bar die Pumpe 11 zu fördern beginnt, so dass das Kabel 10 auf Durchschalten durch den Druckschalter 13 gesteuert ist. Bei einem Druck von unter 1 bar kann die Förderung durch die Pumpe 11 beendet werden.

Wenn der Druck bei 1 bar oder etwas über 1 bar liegt, ist davon auszugehen, dass eine Pumpe in der Reinigungsvorrichtung 40, die in Fig. 1 und auch sonst nicht dargestellt ist, entsprechend ein Fluid umwälzt, so dass klar ist, dass ausreichend Fluid zur Weiterförderung vorhanden ist. Die Pumpe 11 sorgt beispielsweise dafür, dass der Druck, der in dem Reservoir 14 vorherrscht, erhöht wird, so dass am Ausgangsanschluss 5 ein Fluid mit einem bevorzugten Druck von 2 bis 3 bar zur Verfügung gestellt wird.

Alternativ zum Druckschalter 13 kann ein Drucksensor 12 vorgesehen sein, mittels dessen ein nicht dargestellter Schalter im Kabel 10 bzw. in der Leitung 10 geschaltet werden kann. Es kann auch eine Spannungsversorgung anstelle des Akkumulators 8 vorgesehen sein, die variabel ist und je nach vorherrschendem Druck im Reservoir 14 entsprechend eine angepasste Spannung vorsieht, um eine hierdurch vorbestimmbare bzw. vorgebbare Druckerhöhung des Fluids vorzusehen. Hierzu kann durch den Sensor 12 ein Potentiometer angesteuert werden, mittels dessen die der Pumpe 11 zugeführte Spannung variiert werden kann. Alternativ kann ein Drucksensor an dem Ausgangsanschluss 5 vorgesehen sein, mittels dessen die Pumpe 11 gesteuert wird. Die Signale der oder des Drucksensors können auch an die Steuerung der Reinigungsvorrichtung übermittelt werden, so dass der ordnungsgemäße Ablauf des Reinigungsprozesses überwacht werden kann. Alternativ kann ein Indikatorelement, bspw. eine LED, an der Verbindungsvorrichtung vorgesehen sein, um eine Störung im Reinigungsvorgang anzuzeigen.

Das Reservoir 14 hat einen Querschnitt D1, der in Fig. 2 als einfacher Abstand dargestellt ist, der größer ist als der Querschnitt D2 des Zuführanschlusses 4. Hierbei ist der Innenquerschnitt gemeint. Hierdurch kann das Reservoir 14 als eine Art Zwischenspeicher fungieren, so dass eine gleichmäßige und gesicherte Versorgung der Pumpe 11 vorgesehen ist, so dass ohne ungewollte Druckschwankungen der Druck des Fluids erhöht werden kann.

Fig. 3 zeigt eine schematische Draufsicht auf einen erfindungsgemäßen Reinigungsbehälter 70. Das Endoskop 2 ist schon in einen Aufnahmebereich 61 eingebracht. Es sind auch entsprechende Anschlüsse vorgesehen, beispielsweise ein Wasseranschluss 71, der die Aufnahme 61 entsprechend mit Wasser füllt, ein Behandlungsmediumanschluss 72, ein Luft- und/oder Wasseranschluss 73 und ein Absauganschluss 74, der das Wasser aus dem Aufnahmebereich 61 absaugt. Es sind ferner, sofern der Reinigungsbehälter 70 nicht nur in einer Reinigungsvorrichtung 40, sondern auch in einem nicht dargestellten Autoklaven zu verwenden ist, ein Dampfeinlassanschluss 75 an ein Dampfabsauganschluss 76 vorgesehen. Zur besseren Handhabbarkeit sind Handgriffe 77 vorgesehen.

In dem erfindungsgemäßen Reinigungsbehälter 70 ist die erfindungsgemäße Verbindungsvorrichtung 3 schon integriert. Diese kann erfindungsgemäß so integriert sein, dass diese in die Wandungen bzw. in das Gehäuse des Reinigungsbehälters 70 eingebracht ist, so dass der Behandlungsmediumanschluss 72 integral in den Anschluss 4 übergeht oder alternativ kann, was bevorzugt ist, die Verbindungsvorrichtung 3 herausnehmbar aus dem und einbringbar in den Reinigungsbehälter 70 sein. Im zweiten Fall wird dann der Anschluss 4 mit dem Behandlungsmediumanschluss 72 verbunden. Es wird außerdem beispielsweise der Anschluss 5 des Endoskops 2 über einen Schlauch 7 mit dem Ausgangsanschluss 6 der Verbindungsvorrichtung 3 verbunden.

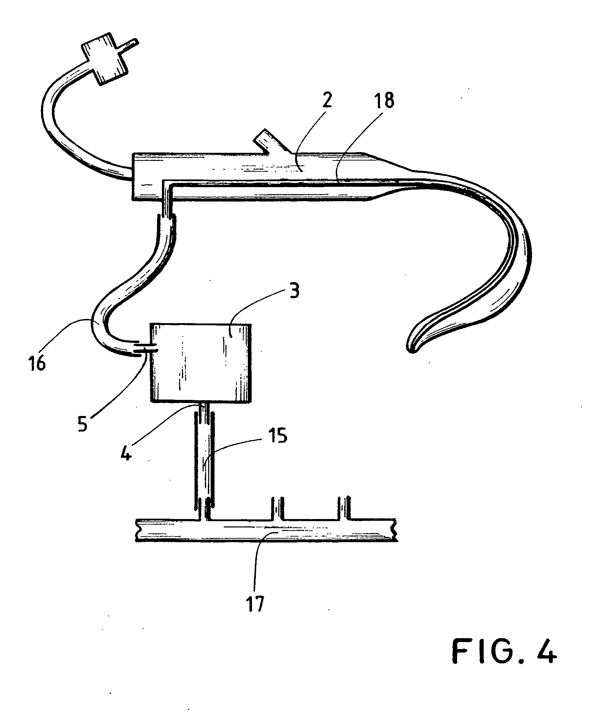

Fig. 4 zeigt schematisch eine Anordnung einer erfindungsgemäßen Verbindungsvorrichtung in einem Reinigungsraum einer Reinigungsvorrichtung. Die Reinigungsvorrichtung weist einen Anschlussverteiler 17 auf, durch den beispielsweise gereinigtes Wasser geführt wird. Ein Anschluss hiervon wird mit einem Schlauch 15 zum Eingangsanschluss 4 der Verbindungsvorrichtung 3 verbunden. Der Ausgangsanschluss 5 der Verbindungsvorrichtung 3 ist über einen Schlauch 16 mit dem Kanal 18 des Endoskops 2 verbunden. Nun kann beispielsweise der Druck, der in dem Anschlussverteiler 17 vorliegt, durch die erfindungsgemäße Verbindungsvorrichtung 3 erhöht werden oder der Fluss des Reinigungsmediums bzw. des flüssigen Mediums, das durch die Verbindungsvorrichtung 3 geführt wird, kann entsprechend gesteuert werden. Es kann auch die Temperatur des durch den Kanal 18 geleiteten Mediums durch eine Heizung, die in der Verbindungsvorrichtung 3 vorgesehen ist, erhöht werden, so dass partiell das Endoskop 2 mit erhöhter Temperatur beaufschlagt wird.

Die Spannungsversorgung kann vorzugsweise wieder aufgeladen werden, so dass die Verbindungsvorrichtung 3 bzw. der Adapter autark gebraucht werden kann.

Die Verbindungsvorrichtung kann nicht nur in einer Reinigungsvorrichtung 40 mit einem oben angeordneten Deckel eingesetzt werden, sondern auch bei einer Reinigungsvorrichtung mit einer frontseitigen Tür. Anstelle der Anordnung der Verbindungsvorrichtung in einem Reinigungsbehälter 35 bzw. 70 kann auch eine erfindungsgemäße Verbindungsvorrichtung in einem Drahtkorb zur Aufnahme wenigstens eines Endoskops Verwendung finden.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 1: Gehäuse
- 2: Endoskop
- 3: Verbindungsvorrichtung
- 4: Anschluss
- 5: Anschluss
- 6: Anschluss
- 7: Schlauch
- 8: Akkumulator
- 9, 10: Kabel
- 11: Pumpe
- 12: Drucksensor
- 13: Druckschalter
- 14: Reservoir
- 15: Schlauch
- 16: Schlauch
- 17: Anschlussverteiler
- 18: Kanal
- 35: Reinigungsbehälter
- 36: Aufnahmebereich
- 37: Aussparung
- 40: Endoskopreinigungsvorrichtung
- 41: Reinigungsbad
- 42: Deckel
- 43: Wasserzuführung
- 44: Desinfektionsmittelzuführung
- 45: Luft- oder Wasserzuführung
- 46: Wasserabsaugung
- 61: Aufnahmebereich
- 70: Reinigungsbehälter
- 71: Wasseranschluss
- 72: Behandlungsmediumsanschluss
- 73: Luft- oder Wasseranschluss
- 74: Absaugungsanschluss
- 75: Dampfeinlassanschluss
- 76: Dampfabsaugungsanschluss
- 77: Handgriff

- D1: Querschnitt
- D2: Querschnitt

## Patentansprüche

1. Verbindungsvorrichtung (3) zur Verwendung in einem Reinigungsraum (36, 41, 61) einer Reinigungsvorrichtung (40, 70) zur Reinigung von chirurgischen Instrumenten (2), insbesondere Endoskopen, wobei die Verbindungsvorrichtung (3) ein Gehäuse (1) mit einem Eingangsanschluss (4) und einem Ausgangsanschluss (5) aufweist, wobei der Eingangsanschluss (4) mit einem Anschluss (44, 72) der Reinigungsvorrichtung (40, 70), mittels dessen ein flüssiges Medium bereitgestellt wird, verbindbar oder verbunden ist, wobei der Ausgangsanschluss (5) mit einem zu reinigenden Kanal (18) des chirurgischen Instruments (2) verbindbar oder verbunden ist, wobei in dem Gehäuse (1) ein Mittel (11) zur Beeinflussung wenigstens einer physikalischen und/oder chemischen Eigenschaft des flüssigen Mediums vorgesehen ist, wobei in dem Gehäuse (1) eine Pumpe (11) zur Veränderung des Drucks des an dem Eingangsanschluss (4) zur Verfügung gestellten Mediums vorgesehen ist, wobei die Pumpe (11) den Druck erhöht, wobei in dem Gehäuse (1) eine autonome Spannungsversorgung (8) vorgesehen ist, um die Pumpe (11) mit Spannung zu versorgen.

2. Verbindungsvorrichtung (3) nach Anspruch 1, **dadurch gekennzeichnet, dass** die Pumpe (11) den Druck auf 1,5 bis 4 bar erhöht.

3. Verbindungsvorrichtung (3) nach Anspruch 1 oder 2, **dadurch gekennzeichnet, dass** die autonome Spannungsversorgung (8) eine Batterie oder ein Akkumulator ist.

4. Verbindungsvorrichtung (3) nach einem der Ansprüche 1 bis 3, **dadurch gekennzeichnet, dass** ein Reservoir (14) für das flüssige Medium in dem Gehäuse (1) stromaufwärts der Pumpe (11) vorgesehen ist.

5. Verbindungsvorrichtung (3) nach Anspruch 4, **dadurch gekennzeichnet, dass** der Querschnitt (D1) des Reservoirs (14) größer als der Querschnitt (D2) des Eingangsanschlusses (4) ist.

6. Verbindungsvorrichtung (3) nach Anspruch 4 oder 5, **dadurch gekennzeichnet, dass** das Reservoir (14) ein Volumen zwischen 100 ml und 10 I aufweist.

7. Verbindungsvorrichtung (3) nach einem der Ansprüche 1 bis 6, **dadurch gekennzeichnet, dass** ein Drucksensor (12, 13) in dem Gehäuse vorgesehen ist.

8. Verbindungsvorrichtung (3) nach Anspruch 7, **dadurch gekennzeichnet, dass** der Drucksensor (12, 13) die Pumpe (11) steuert.

9. Verbindungsvorrichtung (3) nach einem der Ansprüche 1 bis 8, **dadurch gekennzeichnet, dass** das Gehäuse (1) wasserdicht ist.

10. Anordnung mit einer Verbindungsvorrichtung (3) nach einem der Ansprüche 1 bis 9 und einer Reinigungsvorrichtung (40) für chirurgische Instrumente (2), insbesondere Endoskopse, wobei die Verbindungsvorrichtung und in einem Reinigungsraum (41) der Reinigungsvorrichtung angeordnet ist.

11. Anordnung nach Anspruch 10, **dadurch gekennzeichnet, dass** die Verbindungsvorrichtung (3) eingangsseitig mit einem flüssigen Mediumsanschluss (44) der Reinigungsvorrichtung (40) verbunden ist und ausgangsseitig mit einem im Reinigungsraum (41) angeordneten chirurgischen Instrument (2) verbunden ist.

12. Verwendung einer Verbindungsvorrichtung (3) nach einem der Ansprüche 1 bis 9 zum, insbesondere druckerhöhten, Durchleiten eines flüssigen Mediums durch die Verbindungsvorrichtung (3) und Einleiten des, insbesondere druckerhöhten, flüssigen Mediums in ein chirurgisches Instrument (2).

13. Reinigungsbehälter (35, 70) mit einer integrierten Verbindungsvorrichtung (3) nach einem der Ansprüche 1 bis 9.

## Claims

1. A connecting device (3) for use in a cleaning space (36, 41, 61) of a cleaning device (40, 70) for cleaning surgical instruments (2), particularly endoscopes, wherein the connecting device (3) includes a housing (1) with an input connector (4) and an output connector (5), wherein the input connector (4) is connectable or connected to a connector (44, 72) of the cleaning device (40 ,70) by means of which a liquid medium is provided, wherein the output connector (5) is connectable or connected to a passage (18) to be cleaned of the surgical instrument (2), wherein provided in the housing (1) is means (11) for influencing at least one physical and/or chemical property of the liquid medium, wherein provided in the housing (1) there is a pump (11) for altering the pressure of the medium made available at the input connector (4), wherein the pump (11) increases the pressure, wherein provided in the housing (1) there is a self-contained voltage supply (8) in order to supply the pump (11) with voltage.

2. A connecting device as claimed in claim 1, **characterised in that** the pump (11) increases the pressure to 1.5 to 4 bar.

3. A connecting device as claimed in claim 1 or 2, **characterised in that** the self-contained voltage supply (8) is a battery or an accumulator.

4. A connecting device as claimed in one of 1 to 3, **characterised in that** a reservoir (14) for the liquid medium is provided in the housing (1) upstream of the pump (11).

5. A connecting device (3) as claimed in claim 4, **characterised in that** the cross sectional area (D1) of the reservoir (14) is larger than the cross sectional area (D2) of the input connector (4).

6. A connecting device (3) as claimed in claim 4 or 5, **characterised in that** the reservoir (14) has a volume between 100ml and 101.

7. A connecting device (3) as claimed in claims 1 to 6, **characterised in that** a pressure sensor (12, 13) is provided in the housing.

8. A connecting device (3) as claimed in claim 7, **characterised in that** the pressure sensor (12, 13) controls the pump (11).

9. A connecting device (3) as claimed in one of claims 1 to 8, **characterised in that** the housing (1) is watertight.

10. An assembly with a connecting device (3) as claimed in one of claims 1 to 9 and a cleaning device (40) for surgical instruments (2), particularly endoscopes, wherein the connecting device is arranged in a cleaning space (41) of the cleaning device.

11. An assembly as claimed in claim 10, **characterised in that** the connecting device (3) is connected on the input side to a liquid medium connector (44) of the cleaning device (40) and is connected on the output side to a surgical instrument (2) disposed in the cleaning space (41).

12. The use of a connecting device (3) as claimed in one of claims 1 to 9 for conducting a liquid medium, particularly under increased pressure, through the connecting device (3) and introducing the liquid medium, particularly under increased pressure, into a surgical instrument (2).

13. A cleaning container (35, 70) with an integrated connecting device (3) as claimed in one of claims 1 to 9.

## Revendications

1. Système de raccordement (3) destiné à être utilisé dans un espace de nettoyage (36, 41, 61) d'un système de nettoyage (40, 70) pour le nettoyage d'instruments chirurgicaux (2), en particulier d'endoscopes, sachant que le système de raccordement (3) comprend un boîtier (1) pourvu d'un raccord d'entrée (4) et d'un raccord de sortie (5), sachant que le raccord d'entrée (4) peut être raccordé ou est raccordé à un raccord (44, 72) du système de nettoyage (40, 70) au moyen duquel un liquide est fourni, sachant que le raccord de sortie (5) peut être raccordé ou est raccordé à un canal à nettoyer (18) de l'instrument chirurgical (2), sachant qu'on a prévu, dans le boîtier (1), un moyen (11) pour influencer au moins une propriété physique et/ou chimique du liquide, sachant qu'on a prévu, dans le boîtier (1), une pompe (11) destinée à modifier la pression du fluide mis à disposition au niveau du raccord d'entrée (4), sachant que la pompe (11) augmente la pression, sachant qu'on a prévu, dans le boîtier (1), une alimentation en tension autonome (8) pour approvisionner la pompe (11) en tension.

2. Système de raccordement (3) selon la revendication 1, **caractérisé en ce que** la pompe (11) augmente la pression de 1,5 à 4 bars.

3. Système de raccordement (3) selon la revendication 1 ou 2, **caractérisé en ce que** l'alimentation en tension autonome (8) est une batterie ou un accumulateur.

4. Système de raccordement (3) selon l'une quelconque des revendications 1 à 3, **caractérisé en ce qu'**on a prévu un réservoir (14) pour le liquide dans le boîtier (1), en amont de la pompe (11).

5. Système de raccordement (3) selon la revendication 4, **caractérisé en ce que** la coupe transversale (D1) du réservoir (14) est supérieure à la coupe transversale (D2) du raccordement d'entrée (4).

6. Système de raccordement (3) selon la revendication 4 ou 5, **caractérisé en ce que** le réservoir (14) présente un volume compris entre 100 ml et 10 I.

7. Système de raccordement (3) selon l'une quelconque des revendications 1 à 6, **caractérisé en ce qu'**on a prévu un capteur de pression (12, 13) dans le boîtier.

8. Système de raccordement (3) selon la revendication 7, **caractérisé en ce que** le capteur de pression (12, 13) commande la pompe (11).

9. Système de raccordement (3) selon l'une quelconque des revendications 1 à 8, **caractérisé en ce que** le boîtier (1) est imperméable.

10. Agencement comportant un système de raccordement (3) selon l'une quelconque des revendications 1 à 9 et un système de nettoyage (40) pour instruments chirurgicaux (2), en particulier des endoscopes, sachant que le système de raccordement est disposé dans l'espace de nettoyage (41) du système de nettoyage.

11. Agencement selon la revendication 10, **caractérisé en ce que** le système de raccordement (3) est raccordé, côté entrée, à un raccordement pour liquide (44) du système de nettoyage (40) et, côté sortie, à un instrument chirurgical (2) disposé dans l'espace de nettoyage (41).

12. Utilisation d'un système de raccordement (3) selon l'une quelconque des revendications 1 à 9 pour la transmission, en particulier avec augmentation de la pression, d'un liquide à travers le système de raccordement (3) et l'introduction du liquide, en particulier avec augmentation de la pression, dans un instrument chirurgical (2).

13. Récipient de nettoyage (35, 70) pourvu d'un système de raccordement intégré (3) selon l'une quelconque des revendications 1 à 9.
